Europäisches Patentamt

European Patent Office

Office européen des brevets

(11) Veröffentlichungsnummer : **0 520 008 B1**

(12) **EUROPÄISCHE PATENTSCHRIFT**

(45) Veröffentlichungstag der Patentschrift :
**25.01.95 Patentblatt 95/04**

(21) Anmeldenummer : **91906530.0**

(22) Anmeldetag : **08.03.91**

(86) Internationale Anmeldenummer :
**PCT/AT91/00042**

(87) Internationale Veröffentlichungsnummer :
**WO 91/14189 19.09.91 Gazette 91/22**

(51) Int. Cl.⁶ : **G02B 3/10,** G02C 7/06,
A61F 2/16

(54) **VERWENDUNG EINER MULTIFOKALEN DOPPELBRECHENDEN LINSE MIT ANGEPASSTER DOPPELBRECHUNG.**

(30) Priorität : **15.03.90 AT 619/90**

(43) Veröffentlichungstag der Anmeldung :
**30.12.92 Patentblatt 92/53**

(45) Bekanntmachung des Hinweises auf die
Patenterteilung :
**25.01.95 Patentblatt 95/04**

(84) Benannte Vertragsstaaten :
**AT BE CH DE DK ES FR GB GR IT LI LU NL SE**

(56) Entgegenhaltungen :
**EP-A- 0 297 841**
**EP-A- 0 308 705**

(56) Entgegenhaltungen :
**IBM TECHNICAL DISCLOSURE BULLETIN, volume 27, no. 11, April 1985, (New York, US) ;
"Method for aligning the axes of a birefringent
bifocal lens system", pp. 6674-6675
APPLIED OPTICS, volume 8, no. 10, October
1969, R.S. ENG et al. : "Multiple imagery with
birefringent lenses", pp. 2117-2120**

(73) Patentinhaber : **FIALA, Werner**
**Staudgasse 88/11**
**A-1180 Wien (AT)**

(72) Erfinder : **FIALA, Werner**
**Staudgasse 88/11**
**A-1180 Wien (AT)**

(74) Vertreter : **Hofinger, Engelbert, DDr. et al**
**Patentanwälte Torggler & Hofinger**
**Wilhelm-Greil-Strasse 16**
**A-6020 Innsbruck (AT)**

## Beschreibung

Die Erfindung bezieht sich auf die Verwendung einer bifokalen Linse mit einem Linsenkörper aus einem doppelbrechenden Material.

In Fig. 2 der EP-A2 308 705 wird eine doppelbrechende Linsenkomponente gezeigt, die ein Teil eines Linsensystems mit mindestens zwei Linsen ist, wobei mit diesem Linsensystem zwei unabhängig voneinander vorgebbare Brechkräfte bereitgestellt werden können.

Doppelbrechende multifokale Linsensysteme werden auch in der US 4,981,342 beschrieben. Die Linsensysteme dieser Schriften umfassen doppelbrechende und/oder isotrope Linsenkomponenten bzw. Einzellinsen dergestalt, daß zumindest zwei Brechkräften des Linsensystems Werte gegeben werden können, die von den Materialeigenschaften der Komponenten des Linsensystems nicht abhängig sind. Es wird dort gezeigt, daß ein solches Linsensystem aus mindestens einer doppelbrechenden Einzellinse und mindestens einer isotropen oder einer weiteren doppelbrechenden Einzellinse zu bestehen hat, um mindestens zwei Brechkräfte beliebiger vorgegebener Werte bereitstellen zu können. Weiters wird dort gezeigt, daß zumindest zwei doppelbrechende Einzellinsen und eine isotrope oder eine weitere doppelbrechende Einzellinse erforderlich sind, um mindestens drei Brechkräfte beliebiger vorgegebener Werte bereitzustellen. Allgemein wird dort gezeigt, daß bei einem Linsensystem, das M doppelbrechende Einzellinsen umfaßt, die Anzahl $N_{frei}$ der Brechkräfte mit frei vorgegebenen Werten gleich M ist, d.h. $N_{frei} = M$. Es wird in der EP und der US (dortige Gleichungen 23) weiters gezeigt, daß das System um zumindest eine weitere isotrope Einzellinse zu erweitern ist, um eine Anzahl von $N_{frei} = M+1$ Brechkräften bereitzustellen, die beliebige vorgegebene Werte aufweisen.

Aufgabe der Erfindung ist es, eine konstruktiv einfache ophthalmische Linseneinrichtung bereitzustellen, die zwei verschiedene, unabhängig voneinander vorgegebene Brechkräfte $D_{max}$, $D_{min}$ aufweist.

Gemäß der gegenständlichen Erfindung wird dies durch die Merkmale des Anspruches 1 erreicht.

Weiters ist es möglich, die bifokale Linse als Intraokularlinse, die sich in einem Immersionsmedium befindet und die bei Einfall von natürlichem, d.h. unpolarisiertem Licht gleichzeitig mindestens zwei frei auswählbare bzw. erforderliche Brechkräfte aufweist, zu verwenden.

Kurze Beschreibung der Zeichnung:

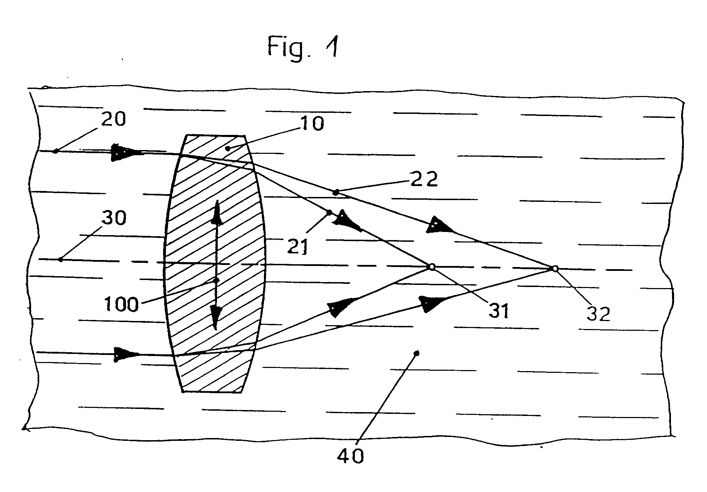

In der Fig. 1 ist eine doppelbrechende Einzellinse 10 gemäß der Erfindung dargestellt, die aus einem doppelbrechenden Material gefertigt ist, dessen Doppelbrechung $\delta n$ durch

$$\delta n = (n_o - n_m)(\frac{D_{max}}{D_{min}} - 1)$$

gegeben ist, wobei $D_{max}$ und $D_{min}$ zwei für eine optische Anwendung gerade erforderlichen Brechkräfte sind. Durch diese Wahl der Doppelbrechung ist erreicht, daß diese Einzellinse die zwei gewünschten, d.h. vorgegebenen Brechkräfte $D_{min}$ und $D_{max}$ besitzt, wenn sie sich in einem Medium 40 mit dem Brechungsindex $n_m$ befindet; die optische Kristallachse 100 der Linse 10 steht senkrecht zur Linsenachse 30; parallel zur Linsenachse 30 einfallende natürliche Lichtstrahlen bzw. -wellen 20 werden von der Linse 10 so gebrochen, daß die resultierenden Lichtstrahlen 21 und 22 entstehen; die Lichtstrahlen 21 werden in den Brennpunkt 31 gelenkt, die Lichtstrahlen 22 werden in den Brennpunkt 32 gelenkt; der Brennpunkt 31 entspricht der Brechkraft $D_{max}$, der Brennpunkt 32 entspricht der Brechkraft $D_{min}$.

Beschreibung der bevorzugten Ausführungsformen:

Die zwei Brechkräfte $D_o$ und $D_e$ einer doppelbrechenden Linse, gemessen in Luft bzw. Vakuum, sind näherungsweise durch

$$D_o = (n_o - 1)S \quad (1)$$

und

$$D_e = (n_e - 1)S$$

gegeben, wobei $D_o$ die Brechkraft der ordentlichen Strahlen und $D_e$ die Brechkraft der außerordentlichen Strahlen ist; $n_o$ ist der ordentliche Brechungsindex, $n_e$ ist der außerordentliche Brechungsindex, S ist der Formfaktor der Linse. Der Formfaktor S ist eine Funktion der geometrischen Parameter der Linse (siehe z.B. J-.Strong: "Concepts of Classical Optics", p. 319; W.H.Freeman and Company, 1958). Der Einfachheit halber kann der Formfaktor S näherungsweise durch

$$S = 1/R_F - 1/R_B \quad (i)$$

dargestellt werden, wobei $R_F$ der Krümmungsradius der vorderen Linsenfläche und $R_B$ der Krümmungsradius der hinteren Linsenfläche ist. Diese Radien haben positive Werte, wenn die zugehörige Linsenfläche bezüglich des einfallenden Lichtes konvex ist; sie sind negativ, wenn die zugehörige Linsenfläche für einfallendes Licht konkav ist.

Wenn sich die Linse in einem Immersionsmedium mit Brechungsindex $n_m$ befindet, dann betragen die Brechkräfte $D_{o,m}$ und $D_{e,m}$ dieser Linse, wobei $D_{o,m}$ mit dem ordentlichen Brechungsindex und $D_{e,m}$ mit dem außerordentlichen Brechungsindex assoziiert wird:

$$D_{o,m} = (n_o - n_m)S \quad (3)$$

und

$$D_{e,m} = (n_e - n_m)S \quad (4)$$

Aus den Gleichungen 1 bis 4 ist sofort abzuleiten, daß die Differenz der Brechkräfte unabhängig vom Brechungsindex $n_m$ des Immersionsmediums ist, d.h.:

$$D_e - D_o = D_{e,m} - D_{o,m} = (n_e - n_o)S \quad (5)$$

Sollen nun die beiden Brechkräfte $D_{o,m}$ und $D_{e,m}$ der doppelbrechenden Linse zwei für spezifische optische Anwendungen notwendige Werte annehmen, so müssen die beiden Indices $n_o$ und $n_e$ des Materials, aus dem die Linse gefertigt ist, die folgende Bedingung erfüllen:

$$n_e = n_m + \frac{D_{e,m}}{D_{o,m}}(n_o - n_m) \quad (6)$$

Ist $D_{o,m}$ die größere, mit $D_{max}$ bezeichnete, und $D_{e,m}$ die kleinere, mit $D_{min}$ bezeichnete der beiden Brechkräfte, so folgt aus der Gleichung 6 die folgende Beziehung:

$$n_e - n_o = (n_o - n_m)(\frac{D_{max}}{D_{min}} - 1) \quad (6.1)$$

Ist hingegen $D_{e,m}$ die größere, mit $D_{max}$ bezeichnete, und $D_{o,m}$ die kleinere, mit $D_{min}$ bezeichnete der beiden Brechkräfte, so folgt aus der Gleichung 6 die Beziehung:

$$n_e - n_o = (n_o - n_m)(\frac{D_{min}}{D_{max}} - 1) \quad (6.2)$$

Im allgemeinen ist es nicht möglich, die Gleichung 6.1 oder 6.2 für beliebig vorgegebene Brechkräfte $D_{max}$ und $D_{min}$ und für beliebiges Immersionsmedium vom Brechungsindex $n_m$ auf der Basis einer doppelbrechenden Linse zu erfüllen, die aus einem doppelbrechenden Material hergestellt ist, das die beiden unabhängig gegebenen Brechungsindices $n_o$ und $n_e$ besitzt. Aus diesem Grund werden in der EP O 308 705 A2 und der US 4,981,342 zumindest zwei Einzellinsen in einem doppelbrechenden Linsensystem vorgesehen, um zwei gewünschte Brechkräfte bereitzustellen.

Wie aber unten gezeigt wird, läßt sich die o.a.Bedingung 6.1 oder 6.2 erfüllen, wenn die doppelbrechende Linse aus einem Linsenmaterial hergestellt wird, das durch einen Orientierungsprozeß, wie Reckung, doppelbrechend gemacht wird, und bei dem verschiedene Orientierungsgrade, die mit verschieden starker Reckung korrelieren, verschieden große Doppelbrechung $\delta n = (n_e - n_o)$ bewirken.

Aus Veröffentlichungen von z.B. Weeger et al. Colloid Polym.Sci.266:692-700(1988) oder J.A.Slee et al.J-.Polym.Sci.Polym.Phys.Vol27,71-80(1989) ist es bekannt, daß die durch Reckung induzierte Doppelbrechung $(n_e-n_o)$ monoton mit dem Reckgrad zunimmt, vorausgesetzt, daß alle anderen Parameter der Reckung konstant gehalten werden. Die induzierte Doppelbrechung hängt auch von vielen anderen Parametern, wie Recktemperatur, Reckgeschwindigkeit, Temperbedingungen etc. ab; es ist aber stets möglich, durch Variation der Reckbedingungen die resultierende induzierte Doppelbrechung zu variieren. Die meisten orientierten Polymere können als ein Gefüge bestehend aus dünnen zylindrischen Stäben aufgefaßt werden. Gemäß Max Born und Emil Wolf: Principles of Optics, Pergamon Press, 6th Ed.p.707 sind solche Medien positiv doppelbrechend, d.h. sie haben zwei Brechungsindices mit $n_e > n_o$.

Ein Polymer, das im ungereckten Zustand des isotropen Brechungsindex $n_{iso}$ besitzt, weist nach Reckung in der Regel einen ordentlichen Brechungsindex $n_o$ auf, der kleiner ist als $n_{iso}$, und einen außerordentlichen Brechungsindex $n_e$, der größer ist als $n_{iso}$. Diese in Reckversuchen bestätigte Eigenschaft kann durch die empirische Beziehung

$$(n_e - n_{iso}):(n_{iso} - n_o) = k \quad (7)$$

ausgedrückt werden, wobei k in der Regel Werte von 1 bis 3 annimmt. Kombiniert man die Beziehungen 5 bis 7 und löst man für $n_e-n_o$, so erhält man:

$$\delta n = (n_e - n_o) = (1 + k)\frac{(D_{e,m} - D_{o,m})(n_{iso} - n_m)}{(D_{e,m} - D_{o,m}) + (1 + k)D_{o,m}} \quad (8)$$

Für das Beispiel einer Intraokularlinse ist die Differenz der gewünschten Brechkräfte $D_{e,m} - D_{o,m}$ gegenüber den Brechkräften $D_{o,m}$ und $D_{e,m}$ selbst klein, d.h.

$$D_{e,m} - D_{o,m} << D_{o,m}(1 + k) \quad (9)$$

Daraus folgt, daß die erforderliche Doppelbrechung $n_e-n_o$ einer Intraokularlinse, die die beiden erwünschten Brechkräfte $D_{o,m}$ und $D_{e,m}$ besitzen soll, größenordnungsmäßig durch

$$\delta n = (n_e - n_o) \leqq (\frac{D_{e,m}}{D_{o,m}} - 1)(n_{iso} - n_m) \quad (10)$$

gegeben ist.

Im Falle einer Intraokularlinse sind unter der Annahme, daß das Linsenmaterial ein positiv doppelbrechen-

3

des Medium ist, als typische Werte zu nennen: $D_{min} = D_{o,m} = 20$ Dioptrien, und $D_{max} = D_{e,m} = 23$ Dioptrien, wobei diese Werte für eine in einem Medium mit dem Brechungsindex von 1.336 befindliche Linse gelten. Damit folgt aus Gleichung 8, daß die erforderliche Doppelbrechung $n_e$-$n_o$ größenordnungsmäßig 0.02 bis 0.04 betragen muß, wenn das ungereckte Polymer einen Brechungsindex von ca. 1.5 bis 1.6 hat; je größer der isotrope Brechungsindex ist, umso größer muß die Doppelbrechung sein.

J.A.Slee, loc.cit., berichtet über Werte der Doppelbrechung zwischen 0.011 und 0.138 an unter verschiedenen Bedingungen gereckten Proben von Polyäthylenteraphthalat (PET). Da der isotrope Brechungsindex von PET ca. 1.58 beträgt, ist klar, daß die erforderliche "angepaßte" Doppelbrechung in Platten aus PET durch uniaxiale Reckung induziert werden kann.

PET ist aber nicht das einzige und nicht das am besten geeignete Material für die hier beabsichtigten Zwecke. Es wurden ca. 2 - 4 mm dicke Platten anderer Kunststoffe bei verschiedenen Temperaturen und mit verschiedenen Reckgraden gereckt; einige Ergebnisse werden hier wiedergegeben:

| Material | Doppelbrechung |
|---|---|
| Polycarbonat | 0.01 - 0.05 |
| Polysulphon | 0.02 - 0.06 |
| Polyethersulphon | 0.01 - 0.08 |
| Polystyrol | 0.01 - 0.03 |
| Polyestercarbonat | 0.02 - 0.035 |

Im besonderen wird als Beispiel angeführt, daß Polycarbonat bei ca. 135°C auf ca. doppelte Originallänge gereckt wurde. Die Brechungsindices - mithilfe eines Refraktometers in polarisiertem Licht gemessen - betrugen nachher: $n_o = 1.57$ und $n_e = 1.606$. Eine aus dieser gereckten Polycarbonatprobe gefertigte Bikonvexlinse mit den sphärischen Krümmungsradien $R_F = 36$ mm und $R_B = -18$ mm wies bei einer Mitteldicke von 0.8 mm die beiden in Kochsalzlösung mit dem Brechungsindex $n_m = 1.336$ gemessenen Brechkräfte 20.2 und 23.3 Dioptrien auf. Das Auflösungsgitter des Brennweitenmeßgerätes konnte in beiden Brennpunkten aufgelöst werden. Geringe Abweichungen der gemessenen Brechkräfte von den theoretisch zu erwartenden sind aller Wahrscheinlichkeit auf die nur näherungsweise Bestimmungen der Brechungsindices mittels eines einfachen Refraktometers zurückzuführen. Die Musterlinse ist schematisch in Fig. 1 wiedergegeben.

Aus dem Gesagten ist es ersichtlich, daß es möglich ist, bifokale Intraokularlinsen mit verschiedenen Kombinationen der Brechkräfte aus Linsenmaterial verschiedener Doppelbrechung herzustellen. Es ist dabei besonders vorteilhaft, daß verschiedenen Doppelbrechungen in Linsenmaterialien identischer chemischer Zusammensetzung durch Variation der Reckbedingungen induziert werden können. Deshalb müssen ggf. notwendige Bio-Kompatibilitätsprüfungen nur für ein Material durchgeführt werden; weiters sind Produktionstechniken für lediglich ein Material zu entwickeln. Produktionstechniken, wie z.B. Drehen, sind praktisch identisch mit jenen für monofokale Linsen, da die erforderlichen geringen Doppelbrechungen eine nur geringe mechanische Anisotropie des Linsenmaterials bedingen; weiters ist die Linsenform der Bifokallinse äquivalent jener einer Monofokallinse, d.h. die Linse besitzt zwei glatte Oberflächen.

Die Vorteile von multifokalen Linsen der doppelbrechenden Art, wie z.B. optimale Intensitätsverteilung, Wegfall von Intensitätsverlusten (z.B. in höheren Ordnungen, vgl. dazu Diffraktionslinsen), Unabhängigkeit der Brechkräfte und der Intensitätsaufteilung von der Apertur der Linse bzw. des einfallenden Lichtes, geringe chromatische Abberation (vgl. dazu Diffraktionslinsen) etc. wurden schon in der EP O 308 705 und der US 4,981,342 angeführt. Es versteht sich von selbst, daß diese Vorteile auch für die doppelbrechenden Linsen gemäß der gegenständlichen Erfindung zutreffen.

## Patentansprüche

1. Verwendung einer bifokalen Linse mit einem Linsenkörper aus einem doppelbrechenden Material, das ein durch einen Orientierungsprozeß, wie unter einstellbaren Bedingungen vorgenommenes Recken, doppelbrechend gemachtes Polymer ist und das nach dem Orientierungsprozeß einen ordentlichen Brechungsindex $n_o$ und einen außerordentlichen Brechungsindex $n_e$ besitzt, wobei die Doppelbrechung des doppelbrechenden Materials durch den Orientierungsprozeß so eingesellt wird, daß für den Wert der Doppelbrechung $\delta n = n_e$-$n_o$ entweder die Gleichung

$$\delta n = (n_o - n_m)(\frac{D_{max}}{D_{min}} - 1)$$

oder die Gleichung

$$\delta n = (n_o - n_m)(\frac{D_{min}}{D_{max}} - 1)$$

gilt, wobei $D_{max}$ und $D_{min}$ die beiden Werte für die Brechkraft sind, die erhalten werden, wenn sich der Linsenkörper in einem Medium mit dem Brechungsindex $n_m$ befindet, als ophthalmische Einzellinse, wobei $D_{max}$ und $D_{min}$ zwei für die spezifische optische Anwendung erforderliche Brechkräfte sind.

2. Verwendung der bifokalen Linse nach Anspruch 1, wobei die Linse als Intraokularlinse verwendet wird.

**Claims**

1. Use of a bi-focal lens with a lens body made from a birefringent material, which is a polymer which is made birefringent by an orientation process, such as elongation performed under regulatable conditions, and which following the orientation process has an ordinary refractive index $n_o$ and an extraordinary refractive index $n_e$, wherein the birefringence of the birefringent material is regulated by the orientation process so that for the birefringency value $\delta n = n_e - n_o$, either the equation

$$\delta n = (n_o - n_m)(\frac{D_{max}}{D_{min}} - 1)$$

or the equation

$$\delta n = (n_o - n_m)(\frac{D_{min}}{D_{max}} - 1)$$

is applicable, wherein $D_{max}$ and $D_{min}$ are the two values for the birefringency which are obtained when the lens body is in a medium with the refractive index $n_m$, as an ophthalmic single lens, wherein $D_{max}$ and $D_{min}$ are two refractive powers required for the specific optical use.

2. Use of the bi-focal lens according to claim 1, wherein the lens is used as an intraocular lens.

**Revendications**

1. Utilisation d'une lentille bifocale présentant un corps de lentille en matériau biréfringent qui est défini par un polymère rendu biréfringent grâce à un processus d'orientation, par exemple un étirage réalisé dans des conditions réglables, et qui possède après le processus d'orientation un indice de réfraction ordinaire $n_o$ et un indice de réfraction extraordinaire $n_e$, la biréfringence du matériau biréfringent étant réglée grâce au processus d'orientation pour que l'on ait, pour la valeur de biréfringence $\delta n = n_e - n_o$, soit l'équation

$$\delta n = (n_o - n_m)(\frac{D_{max}}{D_{min}} - 1),$$

soit l'équation

$$\delta n = (n_o - n_m)(\frac{D_{min}}{D_{max}} - 1),$$

dans lesquelles $D_{max}$ et $D_{min}$ désignent les deux valeurs pour la force de réfraction qui sont obtenues lorsque le corps de lentille se trouve dans un milieu présentant l'indice de réfraction $n_m$, comme lentille individuelle ophtalmique, et $D_{max}$ et $D_{min}$ étant deux forces de réfraction nécessaires pour l'application optique spécifique.

2. Utilisation de la lentille bifocale selon la revendication 1, selon laquelle la lentille est utilisée comme lentille intra-oculaire.

# Fig. 1